# EUROPEAN PATENT APPLICATION

(11) **EP 4 810 051 A1**
(43) Date of publication of application: **23.09.2026**
(21) Application number: 24890810.5
(22) Date of filing: 15.11.2024
(51) Int. Cl.: A61K 36/48, A61P 27/02

(54) **METHOD FOR AMELIORATING DRY EYE SYNDROME USING ETHANOL-DISTILLED PRODUCT OF SOYBEAN SEEDS**

(30) Priority: 15.11.2023 US 202363599449 P
(71) Applicant: Botanicure CO., LTD., Tainan City 744092 (TW)
(72) Inventor: CHU, I-hung, Tainan City Taiwan 744092 (TW); WENG, Chih-yi, Tainan City Taiwan 744092 (TW)
(74) Representative: Mewburn Ellis LLP
(86) International application number: PCT/CN2024/132295
(87) International publication number: WO 2025/103458

(57) **Abstract**

The present invention discloses that an ethanol-distilled product of soybean seeds can be used to ameliorate dry eye syndrome.

## Description

### FIELD

The present invention relates to a method for improving dry eye syndrome using an ethanol-distilled product of soybean seeds (*Glycine max* seed).

### BACKGROUND ART

Dry eye syndrome (DES) is mainly caused by insufficient tear secretion and excessive tear evaporation. The main symptoms of dry eye syndrome include dryness, redness, itching, photophobia and pain. When the symptoms are severe, they may even cause visual disturbance and ocular surface damage.

Dry eye syndrome is mainly treated clinically with artificial tears for mild cases, while severe cases require anti-inflammatory drugs (SAIDs) for treatment. However, artificial tears and anti-inflammatory drugs can only temporarily improve eye discomfort and cannot increase tear secretion.

Soybean (*Glycine max*) is a plant of the genus Glycine in the family Fabaceae. The main part used is the seed, which is considered a good source of protein intake. In TW I640318 B (corresponding to US 10543243 B2), the applicant disclosed an extraction composition, which includes an extraction product obtained by extracting soybean seeds using water or ethanol below 90 wt%, and a distilled product obtained by distilling soybean seeds using water or ethanol below 15 wt%. The extraction composition has been shown to have the effect of promoting skin wound healing and nerve cell proliferation and treating dementia and breast cancer. In TW I724417 B (corresponding to US 11083766 B2), the applicant disclosed the use of the extraction composition in alleviating pain and skin inflammation caused by cancer radiotherapy.

As far as the applicant is aware, there is no literature or patent case that has ever disclosed that a distilled product of soybean seeds can be used to improve dry eye syndrome.

### SUMMARY OF THE INVENTION

In the present invention, the applicant discovered through experiments that an ethanol-distilled product of *Glycine max* seed can effectively increase the tear secretion of mice with scopolamine-induced dry eye syndrome, and is believed to have the effect of improving dry eye syndrome.

Therefore, in a first aspect, the present invention provides a method for improving dry eye, which comprises administering a pharmaceutical product including the ethanol-distilled product of *Glycine max* seed as described above to a subject in need thereof.

In a second aspect, the present invention provides use of the ethanol-distilled product of *Glycine max* seed as described above for preparing a pharmaceutical product for improving dry eye syndrome.

In a third aspect, the present invention provides a pharmaceutical product for improving dry eye, which includes the ethanol-distilled product of *Glycine max* seed as described above.

Preferably, the ethanol-distilled product is obtained by distilling soybean seeds using 0.01 wt% to 15 wt% of ethanol.

Preferably, the ethanol-distilled product is obtained by distilling soybean seeds using 0.13 wt% to 15 wt% of ethanol.

Preferably, the pharmaceutical product further includes a pharmaceutically acceptable carrier.

Preferably, the pharmaceutical product is in a dosage form for oral, topical or parenteral administration.

### BRIEF DESCRIPTION OF THE DRAWINGS

The above and other objects, features and advantages of the present invention will become apparent after referring to the following detailed description and preferred embodiments and the accompanying drawings, in which:

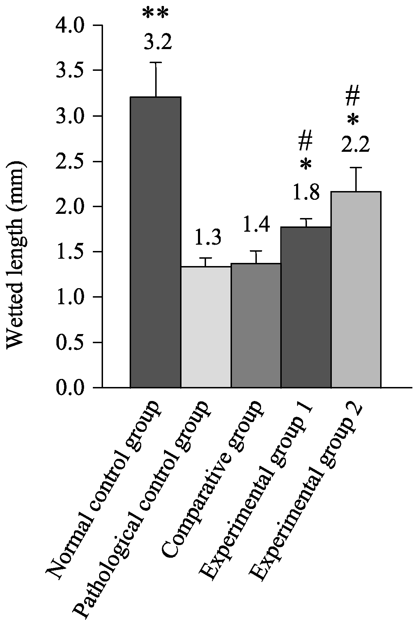

FIG. 1 shows the wetted length measured for each group of mice in Example 1 below, wherein "*" and "**" indicate *p*<0.05 and *p*<0.01, respectively, when compared with the pathological control group; and "#" and "##" indicate p<0.05 and *p*<0.01, respectively, when compared with the comparative group.

### DETAILED DESCRIPTION

For the purposes of this specification, it will be expressly understood that the word "comprising" means "including, but not limited to," and that the word "comprises" has a corresponding meaning.

Unless otherwise defined, all technical and scientific terms used in this article have the meaning commonly understood by those skilled in the art. Those skilled in the art will recognize that many methods and materials similar or equivalent to those described herein can be used to implement the present invention. Of course, the present invention is in no way limited to the methods and materials described.

The present invention provides an ethanol-distilled product of soybean seeds (*Glycine max* seed) for use in preparing a pharmaceutical product for alleviating dry eye syndrome.

In addition, the present invention also provides a pharmaceutical product for alleviating dry eye syndrome, wherein the pharmaceutical product includes the ethanol-distilled product of soybean seeds as described above.

As used herein, the terms "dry eye syndrome," "xerophthalmia," "sclerophthalmia," "keratoconjunctivitis sicca," and "dysfunctional tear syndrome" are used interchangeably and are intended to cover at least one of the following forms: aqueous tear-deficient dry eye, mucin-deficient dry eye, lipid-deficient dry eye, and evaporative dry eye.

Dry eye syndrome to which the present invention is applicable may be caused by a variety of factors, including, but not limited to: a deficiency in the tear-flow system; sleep disorders, such as insomnia and sleep apnea syndrome; the natural aging process, particularly menopause; side effects of medications (e.g., antidepressants, blood pressure medications, Parkinson's medications, antihistamines and birth control pills); diseases that affect tear production capability, such as Sjogren's syndrome, rheumatoid arthritis and collagen vascular disease; wearing of contact lens; smoking; particulate matter (PM), such as PM 2.5; dry climate; insufficient blinking; and structural problems that prevent proper closure of an eyelid.

As used herein, the terms " soybean (*Glycine max*)," "edamame (*Glycine max*)," "yellow soybean (*Glycine max*)," and "soybean" may be used interchangeably and are intended to cover soybeans that are readily available to one skilled in the art or collected from natural sources.

According to the present invention, the methods for ethanol distillation of soybean seeds can be carried out by using the techniques well known and commonly used by those skilled in the art. In this regard, reference can be made to, for example, TW I724417 B (corresponding to US 11083766 B2) and TW I640318 B (corresponding to US 11452753 B2, US 11400128 B2 and US 10543243 B2).

It is understood that the operating conditions of the method for ethanol distillation of soybean seeds will be further varied depending on factors such as the processing method of the soybean seeds and the ratio of the soybean seeds to the ethanol, so as to achieve the best extraction effect. The selection of these operating conditions can be routinely determined by those skilled in the art.

According to the present invention, ethanol distillation of soybean seeds can be performed using fresh soybean seeds, or can be performed using soybean seeds that have been previously processed by a process selected from the group consisting of: drying, grinding, chopping, pulverizing, and combinations thereof.

According to the present invention, the ethanol distillation can be performed using soybean seeds and an ethanol solution in a weight ratio ranging from 1:1 to 1:100. In a preferred embodiment of the present invention, the weight ratio is 1:10.

According to the present invention, the ethanol distillation can be performed by using 0.01 wt% to 15 wt% ethanol. Preferably, the ethanol distillation can be performed by using 0.13 wt% to 15 wt% ethanol. In a preferred embodiment of the present invention, the ethanol distillation is performed by using 0.13 wt% ethanol.

According to the present invention, the ethanol distillation can be carried out at a temperature ranging from 55°C to 98°C. In a preferred embodiment of the present invention, the temperature is 90°C.

According to the present invention, the pharmaceutical product may be in a dosage form suitable for parenteral administration, oral administration or topical administration.

According to the present invention, the pharmaceutical product may further include a pharmaceutically acceptable carrier widely used in drug manufacturing technology. For example, the pharmaceutically acceptable carrier may include one or more agents selected from the following: solvent, buffer, emulsifier, suspending agent, decomposer, disintegrating agent, dispersing agent, binding agent, excipient, stabilizer, chelating agent, diluent, gelling agent, preservative, wetting agent, lubricant, absorption delaying agent, liposome, thickeners and the like. The selection and amount of these reagents are within the professional knowledge and routine skills of those familiar with this technology.

According to the present invention, the pharmaceutical product can be manufactured into a dosage form suitable for parenteral administration (including injection, for example, a sterile aqueous solution or dispersion) using techniques well known to those skilled in the art, and administered by a route selected from the group consisting of: intraperitoneal injection, intrapleural injection, intramuscular injection, intravenous injection, intraarterial injection, intraarticular injection, intrasynovial injection, intrathecal injection, intracranial injection, intraepidermal injection, subcutaneous injection, intradermal injection, intralesional injection, and sublingual administration.

According to the present invention, the pharmaceutical product can be manufactured into a dosage form suitable for oral administration using techniques well known to those skilled in the art, including, but not limited to: sterile powders, tablet, troche, lozenge, pellet, capsule, dispersible powder or granule, solution, suspension, emulsion, syrup, elixir, slurry and the like.

According to the present invention, the pharmaceutical product can be manufactured into an external preparation suitable for topical application to the skin using techniques well known to those skilled in the art, including, but not limited to: emulsion, gel, ointment, cream, patch, liniment, powder, aerosol, spray, lotion, serum, paste, foam, drop, suspension, salve and bandage.

According to the present invention, the external preparation is prepared by mixing the pharmaceutical product of the present invention with a base well known to those skilled in the art.

According to the present invention, the base may contain one or more additives selected from the following: water, alcohols, glycols, hydrocarbons (such as petroleum jelly and white petrolatum), waxes (such as paraffin and yellow wax), preserving agents, antioxidants, surfactants, absorption enhancers, stabilizers, gelling agents (such as Carbopol^{®} 941, microcrystalline cellulose, and carboxymethylcellulose), active agents, humectants, odor absorbers, fragrances, pH adjusting agents, chelating agents, emulsifiers, occlusive agents, emollients, solubilizing agents, penetration enhancers, thickeners, anti-irritants, preservatives, colorants and propellants, etc. The selection and amount of these additives are within the professional qualities and routine skills of those familiar with the technology.

Preferably, the pharmaceutical product is the external preparation containing 1 wt% to 20 wt% thickener and 1 wt% to 10 wt% preservative. In a preferred embodiment of the present invention, the pharmaceutical product is an ointment containing 9.65 wt% thickener and 0.35 wt% preservative.

In a preferred embodiment of the present invention, the thickener is polyacrylic acid (carbomer), glyceryl polyacrylate and hyaluronic acid.

In a preferred embodiment of the present invention, the preservative is benzyl alcohol and chloroxylenol.

The present invention also provides a method for alleviating dry eye syndrome, comprising administering the pharmaceutical product as described above to a subject in need thereof.

As used herein, the terms "administrating" and "administration" may be used interchangeably, and refer to introducing, providing or delivering a predetermined active ingredient to a subject by any appropriate route to perform its intended effect.

As used herein, the term "subject" means any mammal of interest, such as humans, monkeys, cows, sheep, horses, pigs, goats, dogs, cats, mice, and rats.

According to the present invention, the dosage and frequency of administration of the ethanol-distilled product of soybean seeds will vary depending on the following factors: the severity of the disease to be treated, the route of administration, and the age, physical condition and response of the individual to be treated. In general, the ethanol-distilled product of soybean seeds may be administered in the form of a single dose or divided into several doses. For example, the ethanol-distilled product of soybean seeds may be administered twice a day.

The present invention will be further described with reference to the following examples. However, it should be understood that these examples are only for illustration and should not be construed as limitations on the implementation of the present invention.

### <Examples>

### Example 1. Evaluation of efficacy of ethanol-distilled product of soybean seeds (Glycine max seed) in alleviating dry eye syndrome

### Experimental Materials:

### 1. Preparation of ethanol-distilled product of soybean seeds:

The ethanol-distilled product of soybean seeds used in this example was prepared by the method described in the example of TW I640318 B (corresponding to US 10543243 B2).

Briefly, soybean seed powder collected from Tainan City was mixed with 0.13 wt% ethanol at a weight ratio of 1:10, and then distilled at 90°C to collect the condensate, so as to obtain the ethanol-distilled product of soybean seeds.

### 2. Preparation of ointment containing ethanol-distilled product of soybean seeds:

Ointments 1 to 3 used in this example had the formulation shown in Table 1 below. Ointment 2 was prepared according to the following steps: dissolving the ethanol-distilled product of soybean seeds in water and heating to a temperature ranging from 95°C to 100°C, then adding polyacrylic acid (carbomer), glyceryl polyacrylate, and hyaluronic acid and stirring, followed by adding benzyl alcohol and chloroxylenol after the resulting mixture was cooled. In addition, the preparation steps of Ointments 1 and 3 were the same as the preparation steps of Ointment 2, except that no ethanol-distilled product of soybean seeds was added to Ointment 1, and for Ointment 3, the ethanol-distilled product of soybean seeds was not dissolved in water before heating.

**Table 1. Formulation of each ointment**

| | Ointment 1 | Ointment 2 | Ointment 3 |
|---|---|---|---|
| Component | Content (wt%) | | |
| Ethanol-distilled product of soybean seeds | 0 | 20 | 90 |
| Polyacrylic acid | 0.28 | 0.28 | 0.28 |
| Glyceryl polyacrylate | 0.07 | 0.07 | 0.07 |
| Hyaluronic acid | 0.15 | 0.15 | 0.15 |
| Benzyl alcohol | 10 | 9.99 | 9.49 |
| Chloroxylenol | 0.01 | 0.01 | 0.01 |
| Water | 89.49 | 69.5 | 0 |

### 3. Experimental Animals:

The male C57BL/6 mice (8 weeks old, weighing approximately 24 g) used in this example were purchased from BioLasco Taiwan Co., Ltd. All experimental animals were kept in an animal room with 12 hours of light and 12 hours of darkness, maintained at 24-26°C and a relative humidity of 55%-65%, and water and feed were sufficiently supplied. The handling of the experimental animals and all experimental procedure designs conformed to the experimental animal management standards of the Association for Assessment and Accreditation of Laboratory Animal Care, International.

### Experimental methods:

### A. Induction of dry eye syndrome:

First, the mice were randomly divided into 5 groups, including a normal control group (n=4), a pathological control group (n=5), a comparative group (n=4), experimental group 1 (n=5), and experimental group 2 (n=4). Next, the mice of the pathological control group, the comparative group, and experimental groups 1 and 2 were each subjected to subcutaneous injection of scopolamine (Sigma, Cat. No. S0929) (at a dose of 0.5 mg/mouse, formulated in 0.2 mL of physiological saline) twice a day, so as to induce dry eye syndrome. As for the mice of the normal control group, no treatment was performed.

### B. Dry eye syndrome dosage:

On the 1^{st} day after starting the injection of scopolamine, Ointments 1 to 3 were applied to the skin around the eyes of the mice of the comparative group and experimental groups 1 and 2, respectively (all doses were: 50 mg of ointment applied per 1 square centimeter of area), twice a day, lasting for a total of 21 days. As for the mice of the normal control group and the pathological control group, no treatment was performed.

### C. Schirmer tear test

On the 21^{st} day after starting the injection of scopolamine, a 1×40 mm² Schirmer tear test strip (purchased from OPTITECH) was placed in the right lower eyelid of the mice in each group for 30 seconds, and then the wetted length was measured and recorded.

The experimental data thus obtained were expressed as "mean ± standard error of the mean (SEM)" and analyzed by Student's t-test so as to evaluate differences among the groups. Statistical significance is indicated if the thus obtained statistical analysis result was *p*<0.05.

### Results:

FIG. 1 shows the wetted length measured in each group of mice. As shown in FIG. 1, the wetted length of the pathological control group was significantly lower than that of the normal control group, indicating that scopolamine successfully induced dry eye syndrome in mice and caused insufficient tear secretion. Compared with the pathological control group and the comparative group, the wetted lengths of experimental groups 1 and 2 were significantly increased, and the increase became more significant as the content of the ethanol-distilled product of soybean seeds increased. This experimental result shows that the ethanol-distilled product of soybean seeds can increase tear secretion in a dose-related manner. Therefore, the ethanol-distilled product of soybean seeds is considered to have high potential for use in preparing a pharmaceutical product for alleviating dry eye syndrome.

All patents and references cited in this specification are incorporated herein by reference in their entirety. In the event of a conflict, the detailed description (including definitions) of this specification will prevail.

Although the present invention has been described with reference to the above specific embodiments, it is apparent that many modifications and variations can be made without departing from the scope and spirit of the invention. It is therefore intended that the present invention be limited only as indicated in the appended claims.

## Claims

1. Use of an ethanol-distilled product of soybean seeds for preparing a pharmaceutical product for alleviating dry eye syndrome.

2. The use according to claim 1, **characterized in that** the ethanol-distilled product is prepared by distilling the soybean seeds using 0.01 wt% to 15 wt% of ethanol.

3. The use according to claim 1, **characterized in that** the ethanol-distilled product is prepared by distilling the soybean seeds using 0.13 wt% to 15 wt% of ethanol.

4. The use according to claim 1, **characterized in that** the pharmaceutical product further includes a pharmaceutical acceptable carrier.

5. The use according to claim 1, **characterized in that** the pharmaceutical product is in a dosage form for oral administration, topical administration, or parenteral administration.

6. A method for alleviating dry eye syndrome, **characterized in that** the method comprises: administering to a subject in need thereof a pharmaceutical product, the pharmaceutical product including an ethanol-distilled product of soybean seeds.

7. The method according to claim 6, **characterized in that** the ethanol-distilled product is prepared by distilling the soybean seeds using 0.01 wt% to 15 wt% of ethanol.

8. The method according to claim 6, **characterized in that** the ethanol-distilled product is prepared by distilling the soybean seeds using 0.13 wt% to 15 wt% of ethanol.

9. The method according to claim 6, **characterized in that** the pharmaceutical product further includes a pharmaceutically acceptable carrier.

10. The method according to claim 6, **characterized in that** the pharmaceutical product is in a dosage form for oral administration, topical administration, or parenteral administration.

11. A pharmaceutical product for alleviating dry eye syndrome, **characterized in that** the pharmaceutical product includes an ethanol-distilled product of soybean seeds.

12. The pharmaceutical product according to claim 11, **characterized in that** the ethanol-distilled product is prepared by distilling the soybean seeds using 0.01 wt% to 15 wt% of ethanol.

13. The pharmaceutical product according to claim 11, **characterized in that** the ethanol-distilled product is prepared by distilling the soybean seeds using 0.13 wt% to 15 wt% of ethanol.

14. The pharmaceutical product according to claim 11, **characterized in that** the pharmaceutical product further includes a pharmaceutically acceptable carrier.

15. The pharmaceutical product according to claim 11, **characterized in that** the pharmaceutical product is in a dosage form for oral administration, topical administration, or parenteral administration.
